# EUROPEAN PATENT APPLICATION

(11) **EP 2 808 055 A1**
(43) Date of publication of application: **03.12.2014**
(21) Application number: 13740736.7
(22) Date of filing: 11.01.2013
(51) Int. Cl.: A61N 1/36

(54) **SYSTEM FOR TREATING IDIOPATHIC SCOLIOSIS**

(30) Priority: 24.01.2012 ES 201230093
(71) Applicant: Tequir, S.l., 46190 Riba Roja del Turia (ES)
(72) Inventor: BRESO PERIS, María Magdalena, E-46190 Riba-Roja del Turia (Valencia) (ES); BARRIOS PITARQUE, Carlos, E-46190 Riba-Roja del Turia (Valencia) (ES); COSTA SANTOS, Carlos, E-46190 Riba-Roja del Turia (Valencia) (ES)
(74) Representative: ABG Patentes, S.L.
(86) International application number: PCT/ES2013/070004
(87) International publication number: WO 2013/110835

(57) **Abstract**

The present invention relates to a system for treating idiopathic scoliosis characterized in that it comprises a programmable subcutaneous or submuscular device (1) connected by means of wiring (5) to a plurality of sensors (2) which are configured to record electromyographic signals and a plurality of stimulators (3) which are configured to stimulate that part of the deep paraspinal muscles that is affected by the pathology, and wherein said device (1) comprises means for wireless data transmission, such that it may be programmed by means of an external console (4), and wherein muscle stimulation is controlled by control logic that comprises a feedback loop algorithm for adjustment of the stimulation based on the results obtained from the sensors (2) for recording the electromyographic signals and according to a musculoskeletal model, for the purpose of creating a suitable stimulus within a set time range.

## Description

The main object of the present invention is a system for treating idiopathic scoliosis characterized by being capable of treating the deep rotator muscles as a result of electrodes based on the data collected by sensors that are both implanted subcutaneously in a programmable subcutaneous device.

### Background of the Invention

In Europe, about 2-3% of children comprised between 10 and 16 years of age are affected by adolescent idiopathic scoliosis (AIS), which is the most common structural deformity of the spine in children and adolescents and is defined as a curve of less than 10°, measured with a foot x-ray using the Cobb method, with rotation of the vertebral bodies of unknown origin. The deformity has a negative impact on adolescents that can lead to a loss of quality of life and even to psychological disorders.

As a result, about 10% of those patients diagnosed with adolescent idiopathic scoliosis will need some type of treatment because specific therapies except observation are generally not required in growth curves of up to Cobb angle of 25°.

On the other hand, about 0.1% of those patients diagnosed will have to correct adolescent idiopathic scoliosis by means of surgery in an attempt to stop the progressive nature of the deformity because if the scoliosis exceeds a critical threshold, generally considered a Cobb angle of 40°, the risk of health problems as an adult increases considerably, with the loss of quality of life that it entails, pain, etc.

Until now, treatments have been based on using a corset-type body jacket, although over the years attempts have been made to implement without demonstrable effectiveness other types of therapy based on the electrical surface simulation, biofeedback, physical therapy, etc., surgery being the last resort due to complications characteristic of the intervention.

Idiopathic scoliosis (IS) has been unknown for many years; however there is growing evidence that deformation of the spinal column is a musculoskeletal expression of the central nervous system disorder. Some clinical studies have demonstrated abnormalities in controlling balance and proprioception in the patients with scoliosis compared to other healthy patients of the same age and sex.

The disorder interferes with the deep interspinal or paraspinal muscles, inducing an imbalance of forces acting on different vertebral segments. The involvement of the deep interspinal or rotator muscles in the imbalance of the forces of rotation explains the asymmetrical development of scoliosis curves. This imbalance of forces during the period of growth could lead to developing scoliosis, with the rotation of the vertebral bodies towards the convex side due to the tonic predominance of the rotator muscles on the opposite side.

The document *"*Scoliosis treatment in children using a programmable, totally implantable muscle stimulator", by M.A. Herbert and W.P. Bobechko, discloses a treatment for scoliosis based on unilateral stimulation of the paraspinal muscles, such that the contraction thereof counteracts progression of the pathological curve. The system consists of an implantable pulse generator connected to a system of stimulation electrodes. The device is implanted under the skin and its activity is externally programmed by RF once it is implanted. The device is not envisaged to incorporate any feedback system based on the detection of electromyographic signals from the actual patient because three electrodes (two positive electrodes and one negative electrode) are described, so the device does not incorporate any system of sensors for this purpose like the system object of the present invention does.

In turn, the device defined in the article is prepared to act like a mere stimulator and only at night, being implanted in the twelfth rib, while the device proposed herein will be located in an area that does not cause discomfort for the patient in the posterior portion in a subcutaneous or submuscular manner.

Based on the aforementioned article, some inventions relating to implantable devices have been developed to stimulate the muscles of the back to treat various illnesses, among them scoliosis, such as US patent US4026301, for example. Nevertheless, once said documents are studied they can be considered to have significant differences with respect to the invention herein proposed because said documents do not establish recording of the muscle activity on which the patient treatment is based, in addition to not sensing, instead they are mere stimulators. However, in the invention object of the present specification stimulation is produced based on a feedback loop algorithm based on the results obtained from the sensors.

The treatment mentioned in said US patent is based on the impulses being developed by an RF transmitter during patient sleep periods, whereas the device herein described is designed so that the implanted device itself transmits impulses by means of stimulators to the paraspinal muscle continuously depending on the sensing both during the day and at night.

On the other hand, it is known to use electromyographic signals of muscles and other body tissues obtained either by means of transcutaneous or implanted electrodes/sensors to monitor and/or treat various pathologies: chronic pain, monitoring of the spine in spinal column operations, study of joint pathologies, etc. As an example, US patent US4669477 describing a system for the active treatment of bruxism by electrical stimulation is known. The apparatus consists of sensors in the form of electrodes located on or near the mandibular joint. The electrodes detect a signal indicative of jaw clenching, and this signal is transmitted to a signal processor, which produces a jaw muscle stimulation signal in response to a certain level of activity of said muscles, which signal is sent to electrodes in the lower jaw to cause the opening thereof. Nevertheless, the invention proposed herein is developed for a different clinical medical setting (the treatment of scoliosis, not mandibular) and therefore uses a specific electrode for use, being implanted in a long-lasting or permanent manner.

Finally, document US5810747 discloses another invention that uses electromyographic signals in the field of muscle rehabilitation. In this case, the signals collected by means of detection electrodes are sent to a remote processing unit; depending on these signals and other collected parameters derived from position sensors, it establishes a customized set of rehabilitation goals for the patient. However, at no time does it mention stimulators and an operating protocol for treating the pathology based on the mentioned results.

### Description of the Invention

To solve the problems described above, the system for treating idiopathic scoliosis, object of the present specification is described, characterized by being a minimally invasive system that is permanently implanted like a muscle simulator for effectively treating idiopathic scoliosis.

The system consists of a programmable subcutaneous device capable of detecting and stimulating the muscles that are most predominant in scoliosis (deep interspinal or paraspinal muscles) as a result of a series of stimulators together with a plurality of sensors (programmed with a software based on a feedback loop algorithm for automatic stimulus fine-tuning), which effectively treat the spinal column deformity.

Implantation thereof is a simple procedure which offers greater patient comfort than those procedures obtained as a result of current solutions, while at the same time enabling better treatment for idiopathic scoliosis by means of using a minimally invasive surgical technique by making an incision of less than about 50 mm in length.

The system itself is made up of a plurality of sensors and stimulators that are integrated in a device and will be implanted under the skin. The purpose of such sensors and stimulators will, on one hand, be to sense the side having correct muscle tone, and to stimulate the side affected by the pathology, depending on the sensing, to achieve high efficiency in correcting the tone lost in the most predominant muscle in scoliosis.

This is all regulated by means of software based on an optimal stimulation protocol, taking into account the behaviour of the musculoskeletal model implemented through a multiple feedback input loop at different levels in the curved vertebral column.

Doctors today have understood the usefulness of neuromuscular electrical stimulation because it has been applied in other medical situations. However, it was unviable for treating scoliosis due to the technological limitations for achieving small implantable units with power levels suitable for real time processing that are necessary for treating deep rotator muscles.

As a result, a more profound characterization of the behavior of muscles in the electrical stimulation threshold for treating scoliosis must be achieved in order to define the loop ratio between the active stimulus of the rotator muscles of one side of the vertebral column and the recorded signal from the opposite muscles on the other side.

The system is based on the concept of trying to correct scoliosis through the stimulus created as a result of a multiple feedback loop input (as a result of a plurality of sensors and stimulators placed on either side of the vertebral column) which allows using a customized stimulation protocol clinically applied in the muscle that is key in scoliosis.

Throughout the description and the claims, the word "comprises" and its variants do not seek to exclude other technical features, additives, components or steps. For persons skilled in the art, other objects, advantages and features of the invention will be understood in part based on the description and in part based on the practice of the invention. The following examples and drawings are provided by way of illustration and are not intended to be limiting of the present invention. Furthermore, the present invention covers all the possible combinations of particular and preferred embodiments herein indicated.

### Brief Description of the Drawings

A series of drawings that aid in better understanding the invention are described very briefly below and expressly refer to an embodiment of said invention described as a non-limiting example thereof.

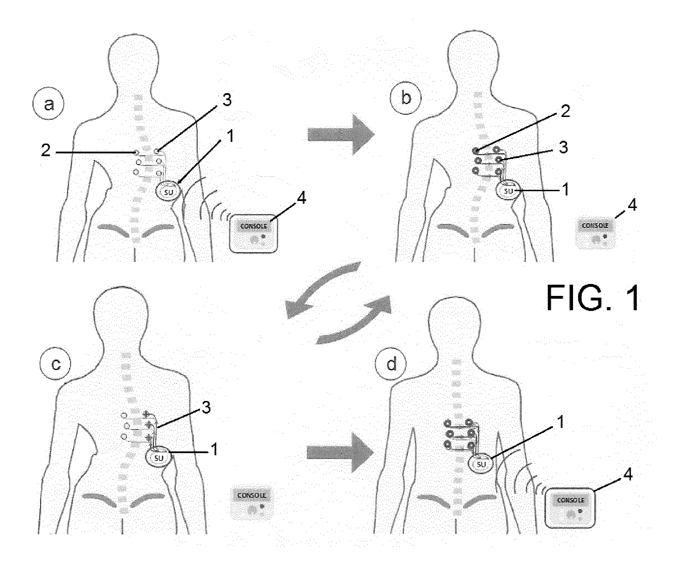

Figure 1 shows a schematic view of the working method of the system for treating idiopathic scoliosis, wherein Figure 1A shows communication while programming the device; Figure 1B shows how the signal is acquired by means of the sensors; Figure 1C shows the electrical stimulation; and Figure 4D schematically shows communication while maintaining and controlling the system.

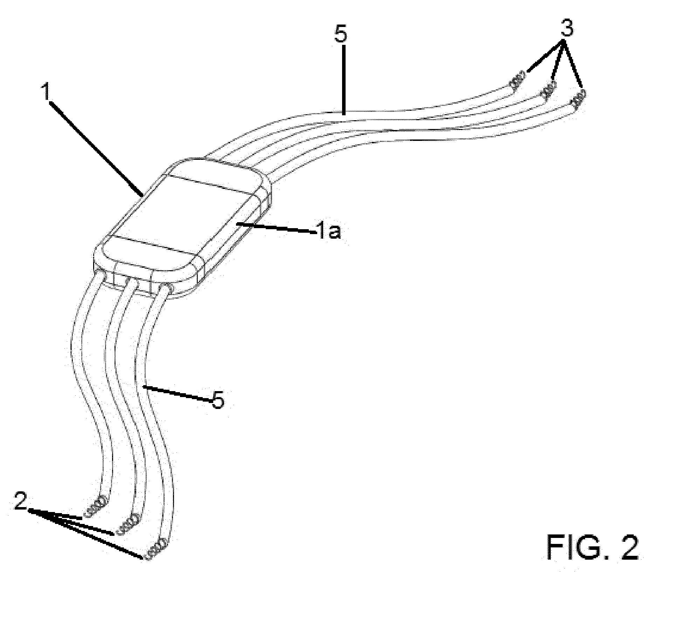

Figure 2 shows a view of a practical embodiment of the implantable subcutaneous device as part of the system for treating idiopathic scoliosis.

### Preferred Embodiment of the Invention

The attached drawings show a preferred embodiment of the invention. More specifically, the system for treating idiopathic scoliosis is characterized in that it comprises a programmable subcutaneous device (1) working in a maximum sensing range of ± 5 mV, formed essentially by a casing (1a).

Connected to said device (1) are a plurality of sensors (2) for recording electromyographic signals, and a plurality of stimulators (3) configured to stimulate that part of the deep paraspinal muscles that is affected by the pathology; both being attached by means of wiring (5) to said subcutaneous device (1).

Muscle stimulation through the stimulators (3) is controlled by control software based on a feedback loop algorithm for adjustment of the stimulation based on the results obtained from the sensors (2) according to a musculoskeletal model for the purpose of creating a suitable stimulus within about 5*10⁻¹ seconds from the acquisition of the electromyographic signals.

To power said device (1), it will have inserted inside the casing (1a) a small energy source in accordance with the power requirements of the system (minimum control circuit voltage: 2.2 V, pulse range of 15 to 40 J).

It is additionally disclosed an external console (4) designed on the basis of RFID operation or on the basis of another wireless data transmission system, and the function of which is to program the subcutaneous device (1) and compile the patient's bioelectrical data. For operation, the RFID tag (working in ranges of 125 kHz) for wireless communication with the subcutaneous device (1) and the control software for selecting the stimulation protocol are "matched up". The external console (4) will always be the responsibility of the doctors responsible for supervising treatment and will be accessible by means of a completely intuitive user interface.

The sensors (2) are configured to be able to measure responses of between 20 Hz and 5 kHz, with an amplifier sensitivity of 50 µV and a scanning duration of about 10 ms.

The stimulators (3) are configured to provide adjustable pulse strength to stimulate the deep paravertebral rotator muscles, with a variable stimulus of 1 to 50 Hz for 1 s, and an adjustable intensity between 0.2 and 50 mA, for a monophasic stimulation pulse.

The number of both sensors (2) and stimulators (3) will vary depending on the levels of spinal column affected by idiopathic scoliosis.

The device (1) will be inserted in the patient by means of the use of minimally invasive surgery to implant the subcutaneous device in the anatomical location through an incision in the skin of less than 50 mm in length and to connect the electrodes to the key muscles through incisions of less than 5 mm in diameter.

## Claims

1. A system for treating idiopathic scoliosis, **characterized in that** it comprises:
a programmable subcutaneous or submuscular device (1) working in a maximum sensing range ± 5 mV, formed essentially by a casing (1a), and electrically powered by means of an energy source; wherein said programmable subcutaneous device is connected by means of wiring (5) to a plurality of sensors (2) which are configured to record electromyographic signals and a plurality of stimulators (3) which are configured to stimulate that part of the deep paraspinal muscles that is affected by the pathology and are located in the deep paraspinal muscles that are both affected by the pathology and not affected by the pathology;
wherein said programmable subcutaneous device (1) comprises means for wireless data transmission, such that it may be programmed by means of an external console (4) by means of RFID or any other form for wireless data transmission, that allows compiling data stored in the device (1) about the sensing and the stimulation, and programming optimal values for each patient depending on the affected levels and the degree of progression of the pathology;
and wherein muscle stimulation is controlled by control logic that comprises a feedback loop algorithm for adjustment of the stimulation based on the results obtained from the sensors (2) for recording the electromyographic signals, and according to a musculoskeletal model, for the purpose of creating a suitable stimulus within a set time range.

2. The system according to claim 1, wherein the external console (4) comprises an RFID tag for wireless communication with the subcutaneous device (1) and control logic means configured to select the stimulation protocol.

3. The system according to claim 2, wherein the external console (4) is accessible by means of a user interface used in clinical use.

4. The system according to claim 1, wherein the sensors (2) are configured to be able to measure responses between 20 Hz and 5 kHz, with an amplifier sensitivity of 50 µV, and with an approximate scanning duration of 10 ms.

5. The system according to claim 1, wherein the stimulators (3) are configured to provide adjustable pulse strength to stimulate the deep paravertebral rotator muscles, with a variable stimulus of 1 to 50 Hz for 1 s, and an adjustable intensity between 0.2 and 50 mA, for a monophasic stimulation pulse.

6. The system according to claim 1, wherein the number of sensors (2) and of stimulators (3) will vary depending on the levels of spinal column affected by idiopathic scoliosis.

7. The system according to claim 1, wherein the set time range to create a stimulus depending on the electromyographic signals of the sensors (2) is comprised in about 5*10⁻¹ seconds from the acquisition of the electromyographic signals.
